# EUROPÄISCHE PATENTSCHRIFT

(11) **EP 1 488 749 B1**
(45) Veröffentlichungstag und Bekanntmachung des Hinweises auf die Patenterteilung: **12.12.2007**
(21) Anmeldenummer: 04011730.1
(22) Anmeldetag: 18.05.2004
(51) Int. Cl.: A61B 17/28

(54) **Medizinisches Instrument**
Medical instrument
Dispositif médical

(30) Priorität: 20.06.2003 DE 10327655
(43) Veröffentlichungstag der Anmeldung: 22.12.2004
(73) Patentinhaber: Karl Storz GmbH & Co. KG, 78532 Tuttlingen (DE)
(72) Erfinder: Lang, Dieter, 96342 Stockheim (DE)
(74) Vertreter: Hofmeister, Frank

(56) Entgegenhaltungen:
- EP-A- 1 250 891
- DE-U- 9 405 468

## Beschreibung

Die Erfindung betrifft ein medizinisches Instrument mit einem hohlen Schaft, an dessen proximalem Ende eine aus mindestens zwei Griffteilen bestehende Handhabe angeordnet ist und an dessen distalem Ende ein aus mindestens zwei Maulteilen bestehendes Werkzeug angeordnet ist, dessen Maulteile des Werkzeugs seitlich abgewinkelt zur Längsachse des Schaftes ausgerichtet sind, wobei mindestens ein Maulteil des Werkzeugs zum Öffnen und Schließen über ein verschwenkbar ausgebildetes Griffteil der Handhabe gegenüber dem mindestens einen anderen Maulteil des Werkzeugs verstellbar ist und das mindestens eine verstellbare Maulteil und das entsprechende, zum Verstellen des Maulteils dienende Griffteil der Handhabe über ein in dem hohlen Schaft gelagertes Zug-/Druckelement miteinander verbunden sind, wobei das Zug-/Druckelement und das jeweilige Maulteil des Werkzeugs über einen Übertragungshebel mit ein auder verbunden sind.

Gattungsgemäße medizinische Instrumente, bei denen das bewegliche Maulteil ausgehend von einem verschwenkbaren Griffteil der Handhabe über ein Zug-/Druckelement betätigbar ist, finden insbesondere in der endoskopischen Chirurgie vielseitige Verwendung, beispielsweise als Stanzen, Scheren, Nadelhalter, Fassinstrumente und dergleichen. Bei den meisten bekannten Instrumenten sind die das Werkzeug bildenden Maulteile in Längsrichtung des Instrumentenschaftes angeordnet, so dass mit diesen Instrumenten nur Operationsgebiete erreichbar sind, die direkt zugänglich im wesentlichen vor der Instrumentenspitze angeordnet sind. Da aber manche Operationsgebiete nicht auf direktem geradlinigen Weg zugänglich sind, sind diese bekannten Instrumente für diese Zwecke gar nicht, oder nur bedingt geeignet.

Um auch abseits der Instrumentenlängsachse angeordnete Operationsgebiete sicher und genau erreichen zu können, sind weiterhin medizinische Instrumente bekannt, deren Maulteile des Werkzeugs seitlich abgewinkelt zur Längsachse des Schaftes ausgerichtet sind. Ein solches gattungsgemäßes Instrument ist beispielsweise aus der DE 91 15 760 U1 bekannt.

Bei diesem bekannten medizinischen Instrument handelt es sich um eine medizinische Zange deren Maulteile so ausgebildet und angeordnet sind, dass sich die Maulteile zum patientenfernen Ende hin öffnen. Hierzu ist das starre Maulteil hakenförmig zurückgebogen ausgebildet, so dass der sich zwischen dem starren Maulteil und dem Instrumentenschaft ergebende Öffnungswinkel zum proximalen Ende der Zange hin gerichtet ist. Das bewegliche Maulteil wird von oben in einen Schlitz des Gehäuses des starren Maulteils eingesetzt, wobei die Verbindung zwischen dem beweglichen Maulteil und der Betätigungsstange über eine in eine Bohrung dieses Maulteils einsetzbare Scheibe erfolgt, in die die Betätigungsstange eingehängt wird.

Sowohl die Lagerung der Scheibe im beweglichen Maulteil als auch die Lagerung des beweglichen Maulteils starren Maulteilgehäuse sind mit Spiel ausgeführt, damit eine Bewegung der Betätigungsstange gegenüber dem beweglichen Maulteil zusammen mit der Scheibe möglich ist. Diese bekannte medizinische Zange weist den konstruktiven Nachteil auf, dass die Kopplung zwischen der Betätigungsstange und dem beweglichen Maulteil an verschiedenen Lagerstellen mit Spiel ausgebildet ist, so dass die Verlagerung der Betätigungsstange über das Griffelement nicht unmittelbar und verzögerungsfrei zur Betätigung des beweglichen Maulteils führt. Aufgrund dieses Spiels ist es für den Operateur nicht möglich, die Zange kontrolliert und dosiert zu betätigen, da zunächst das vorhandene Spielmaß überwunden werden muss, bevor die Zange greift. Ein Instrument mit den Merkmalen des einführenden Teils der Ansprüche 1 und 6 ist in der EP-A-1 250 891 gezeigt.

Davon ausgehend liegt der Erfindung die **Aufgabe** zugrunde, ein medizinisches Instrument der eingangs genannten Art so auszugestalten, dass eine spielfreie und genaue Kraftübertragung auf das Werkzeug gewährleistet.

Die. **Lösung** dieser Aufgabenstellung ist erfindungsgemäß dadurch gekennzeichnet, dass der Übertragungshebel als zweiseitig verdrehbar gelagerter Übertragungshebel ausgebildet ist, der an beiden freien Enden Gelenkkugeln aufweist, die jeweils in entsprechenden Kugelaufnahmen am verstellbaren Maulteil und am Zug-/Druckelement gelagert sind. Eine alternative lösung dieser Aufgabenstellung ist erfindungsgemäß dadurch gekennzeichnet, dass der Übertragungshebel als fest mit dem verstellbaren Maulteil oder fest mit dem Zug-/Druckelement verbundener Stift ausgebildet ist, der in eine entsprechende Führungsbahn im jeweils anderen Bauteil, dem Zug-/Druckelement oder dem verstellbaren Maulteil, eingreift. Durch die erfindungsgemäße Kopplung des Zug-/Druckelements mit dem zu betätigenden Maulteil über einen zwischengeschalteten Übertragungshebel wird eine jederzeit spielfreie und somit direkte Kraftübertragung der über die Handhabe ausgeübten Kraft auf das bewegliche Maulteil gewährleistet.

Die direkte Kraftübertragung ermöglicht dem Operateur einen gezielten und dosierten Krafteinsatz. Statt zunächst einen spielraumbedingten Totraum überwinden zu müssen, bewirkt die Betätigung der Handhabe nunmehr ein unmittelbare Reaktion des Maulteils. Durch entsprechende Ausgestaltung der jeweiligen Übertragungshebel ist es sogar möglich, die zu übertragenden Kräfte funktionsbedingt aufzuteilen und so dem Operateur ein noch besseres Schneid- und Greifgefühl zu geben.

Zur Vermeidung eines Totpunktes bei der Kraftübertragung vom Zug-/Druckelement auf das verstellbare Maulteil ist der Übertragungshebel in Richtung auf das Werkzeug abgewinkelt ausgerichtet.

Gemäß einer ersten erfindungsgemäßen Ausführungsform ist der Übertragungshebel als zweiseitig verdrehbar gelagerter Übertragungshebel ausgebildet, dessen beide freien Enden Gelenkkugeln aufweisen, die jeweils in entsprechenden Kugelaufnahmen am verstellbaren Maulteil bzw. am Zug-/Druckelement gelagert sind.

Mit einer bevorzugten Ausführungsform der Erfindung wird vorgeschlagen, dass die am verstellbaren Maulteil gelagerte Gelenkkugel und/oder die am Zug-/Druckelement gelagerte Gelenkkugel aus zwei Halbkugeln mit unterschiedlichen Durchmessern besteht. Durch die Verwendung der beiden unterschiedlich großen Kugelhälften ist es möglich, die zu übertragenden Kräfte, wie beispielsweise die Kraft zum bloßen Öffnen des Maulteils und die vom Maulteil aufzubringenden Schneid-, Stanz- oder Haltekräfte, auf die unterschiedlichen Kugelhälften aufzuteilen. Neben der genauen Dimensionierung der Halbkugelgrößen an die zu übertragenden Kräfte gewährleistet dies dem Operateur ein gutes und genaues Schnittgefühl.

Zur Ausbildung der Kugelaufnahmen am Maulteil und am Zug-/Druckelement wird erfindungsgemäß vorgeschlagen, dass diese im wesentlichen aus in die jeweiligen Bauteile einsetzbaren Lagerelementen bestehen, nämlich aus einem oder zwei in das verstellbare Maulteil einsetzbaren Lagerelementen, wobei eins der Lagerelemente zum Befestigen der Gelenkkugel in der Aufnahme dient und das gegebenenfalls andere Lagerelement zum Festlegen einer Achse dient, um die sich das Maulteil dreht, sowie einem in das Zug-/Druckelement einsetzbaren Lagerelement. Die Verwendung dieser einsetzbaren Lagerelemente hat den Vorteil, dass die Lagerelemente und auch der Übertragungshebel zu Wartungs- und Reparaturzwecken leicht zugänglich sind.

Weitere Merkmale und Vorteile der Erfindung ergeben sich anhand der Beschreibung der zugehörigen Zeichnung, in der zwei Ausführungsbeispiele eines erfindungsgemäßen medizinischen Instruments nur beispielhaft schematisch dargestellt sind. In der Zeichnung zeigt:
- Fig. 1: eine Seitenansicht einer ersten Ausführungsform eines erfindungsgemäßen medizinischen Instruments, die Maulteile im geschlossenen Zustand darstellend;
- Fig. 2: eine Fig.1 entsprechende Ansicht, jedoch mit geöffneten Maulteilen;
- Fig. 3: einen vergrößerten Schnitt entlang der Linie III-III gemäß Fig. 1;
- Fig. 4: einen vergrößerten Schnitt entlang der Linie IV-IV gemäß Fig. 2;
- Fig. 5: einen vergrößerten Schnitt entlang der Linie V-V gemäß Fig. 2;
- Fig. 6: eine perspektivische ausschnittweise Ansicht des distalen Endes des mit einem Übertragungshebel ausgestatteten Zug-/Druckelements;
- Fig. 7: eine Seitenansicht eines Übertragungshebels und
- Fig. 8: eine ausschnittweise Explosionszeichnung einer zweiten Ausführungsform eines erfindungsgemäßen medizinischen Instruments.

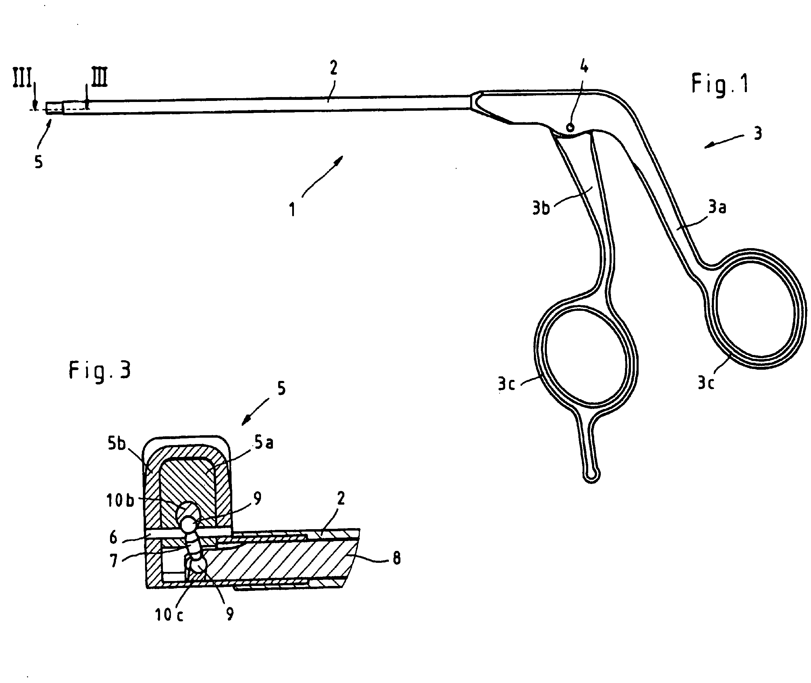

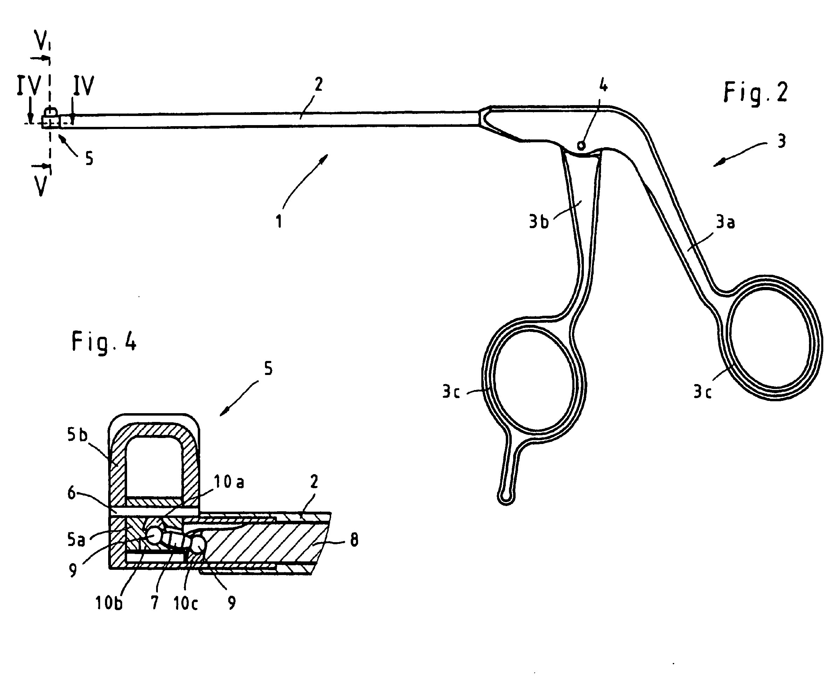

Die Abbildungen Fig. 1 und 2 zeigen Seitenansichten eines medizinischen Instruments 1, dessen Kraftübertragungsmechanismus vielseitig verwendet werden kann, wie beispielsweise für Stanzen, Scheren, Nadelhalter, Fassinstrumente und dergleichen.

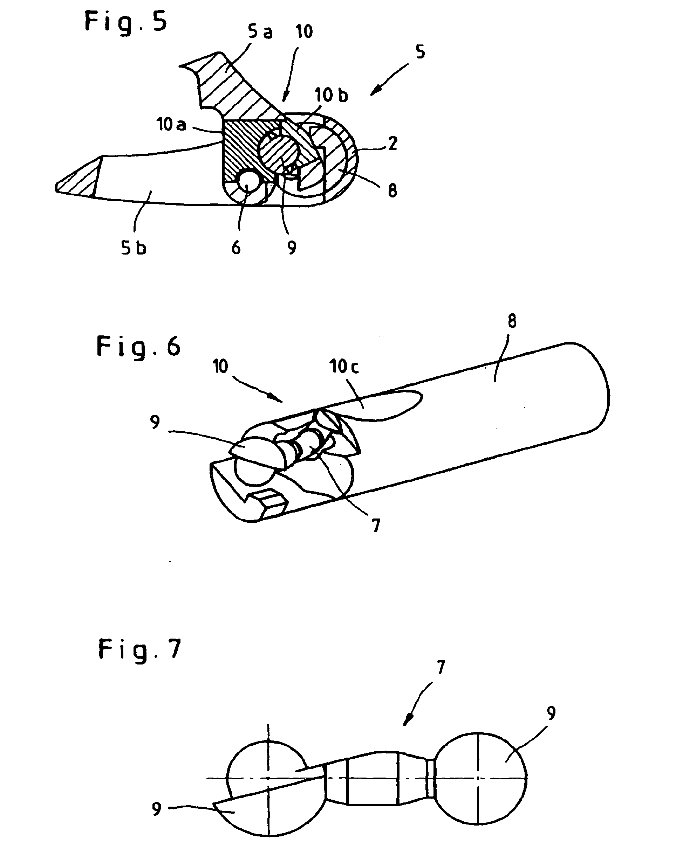

Das medizinische Instrument 1 besteht im wesentlichen aus einem hohlen Schaft 2, an dessen proximalem Ende eine aus einem starren Griffteil 3a und einem gegenüber dem starren Griffteil 3a um eine Schwenkachse 4 verschwenkbaren Griffteil 3b bestehende Handhabe 3 angeordnet ist. Am distalen Ende des Schaftes 2 ist ein Werkzeug 5 angeordnet, welches beim dargestellten Ausführungsbeispiel (Fig. 5) aus einem verschwenkbaren Maulteil 5a und einem starr mit dem Schaft 2 verbundenen Maulteil 5b besteht, wobei das verschwenkbare Maulteil 5a um eine Schwenkachse 6 gegenüber dem starren Maulteil 5b verschwenkbar ist.

Wie aus den Schnittdarstellungen der Abbildungen Fig. 3 und 4 ersichtlich, sind die Maulteile 5a, 5b des Werkzeugs 5 bezüglich der Längsachse des Schaftes 2 seitlich abgewinkelt ausgebildet. Bei den in den Abbildungen Fig. 3, 4 und 8 dargestellten Ausführungsbeispielen sind die Maulteile 5a, 5b jeweils rechtwinklig zur Längsachse des Instrumentenschaftes ausgerichtet. Andere Winkellagen zwischen 0° und 90° und auch darüber hinaus sind jedoch auch praktikabel.

Durch die Abwinklung des Werkzeugs 5 gegenüber dem Schaft 2 ist es möglich, mit einem solchermaßen ausgebildeten medizinisches Instrument 1 auch schwer und nicht auf einem direkten geradlinigen Weg zugängliche Operationsgebiete zu erreichen.

Die Schnittdarstellungen der Abbildungen Fig. 3 und 4 zeigen die Besonderheit des dargestellten medizinischen Instruments 1, nämlich die Verbindung des verschwenkbaren Maulteils 5a über einen Übertragungshebel 7 mit einem Zug-/Druckelement 8, welches in dem hohlen Schaft 2 gelagert proximalseitig so mit dem verschwenkbaren Griffteil 3b der Handhabe 3 verbunden ist, dass durch das Verschwenken des Griffteils 3b das verschwenkbare Maulteil 5a von der geschlossenen Stellung (Fig. 1 und 3) in die geöffnete Stellung (Fig. 2 und 4) bzw. umgekehrt überführt werden kann. Vorteilhafterweise ist das Zug-/Druckelement 8 als Zug-/Druckstange ausgebildet.

Der bei dieser Ausführungsform verwendete Übertragungshebel 7 dient zur Umwandlung und Übertragung der durch das Zug-/Druckelement 8 vollzogenen translatorischen Bewegung in Längsrichtung des Schaftes 2 in die Verschwenkbewegung des rechtwinklig zum Schaft 2 angeordneten verschwenkbaren Maulteils 5a. Wie insbesondere aus Fig. 7 ersichtlich, weist der Übertragungshebel 7 an beiden freien Enden Gelenkkugeln 9 auf, über die der Übertragungshebel 8 in entsprechenden Kugelaufnahmen 10 am verstellbaren Maulteil 5a und am Zug-/Druckelement 8 gelagert ist.

Bei dem dargestellten Ausführungsbeispiel eines Übertragungshebels 7 ist die distalseitige, am verschwenkbaren Maulteil 5a gelagerte Gelenkkugel 9 aus zwei Halbkugeln bestehend ausgebildet, wobei beide Halbkugeln einen unterschiedlichen Durchmesser aufweisen. Bei dieser Anordnung dient die Halbkugel mit dem kleineren Durchmesser vorrangig zur Ausübung der Stanz- oder Schneidkräfte des verstellbaren Maulteils 5a, während die Halbkugel mit dem größeren Durchmesser vorrangig zur Übertragung der vom Zug-/Druckelement 8 ausgeübten Kräfte zum Öffnen des Werkzeugs 5 dient. Durch das Variieren der Halbkugeldurchmesser lässt sich bei im wesentlichen gleichbleibendem Abstand zwischen dem proximalen Ende und dem distalen Ende des Übertragungshebels 7 der Abstand zwischen den beiden Halbkugelmittelpunkten vergrößern, was sich wiederum auf die, zur Vereinfachung der Berechnung, gedachten Hebelverhältnisse und entsprechend der zu übertragenden Kräfte auswirkt. Sogar der tatsächliche, durch den Übertragungshebel 7 gebildete Hebelarm, das heißt der Abstand zwischen dem proximalen Ende und dem distalen Ende des Übertragungshebels 7, lässt sich durch das Variieren der Durchmesser der Halbkugeln der proximalen und distalen Gelenkkugeln 9 vergrößern oder verkleinern.

Die Ausbildung der kleineren Halbkugel zum Ausüben der Stanz- oder Schneidkräfte hat zudem den Vorteil, dass sie beim Zurückziehen des Zug-/Druckelements 8, dem Öffnen des Maulteils 5a, die kraftausübende größere Halbkugel besser vor dem Herausspringen aus der Kugelaufnahme 10 schützt.

Neben der dargestellten Ausführungsform mit der aus unterschiedlich großen Halbkugeln gebildeten Gelenkkugel 9 am distalen Ende des Übertragungshebels 8 ist es selbstverständlich auch möglich, alternativ oder zusätzlich die proximalseitige, am Zug-/Druckelement 8 gelagerte Gelenkkugel 9 aus zwei unterschiedlich großen Halbkugeln bestehend auszubilden. Auch besteht die Möglichkeit, die die Stanz- oder Schneidkräfte übertragende Halbkugel als die Halbkugel mit dem größeren Durchmesser auszubilden. Durch diese Ausgestaltung lässt sich der Hebelarm des gesamten Übertragungshebels 7, nämlich der Abstand zwischen dem distalen Kugelrand der distalen Gelenkkugel 9 und dem proximalen Kugelrand der proximalen Gelenkkugel 9, vergrößern, wobei die Materialstärke der Kugelaufnahmen 10 bei abnehmendem Halbkugeldurchmesser verringert werden kann.

Um den Übertragungshebel 7 einfach montieren zu können, sind die Kugelaufnahmen 10 zur Lagerung der Gelenkkugeln 9 als in das verstellbare Maulteil 5a und das Zug-/Druckelement 8 einsetzbare Lagerelemente 10a, 10b und 10c ausgebildet. Bei der in den Abbildungen Fig. 5 und 6 dargestellten Ausführungsform sind dies zwei Lagerelemente 10a und 10b am verstellbaren Maulteil 5a und ein Lagerelement 10c am Zug-/Druckelement 8.

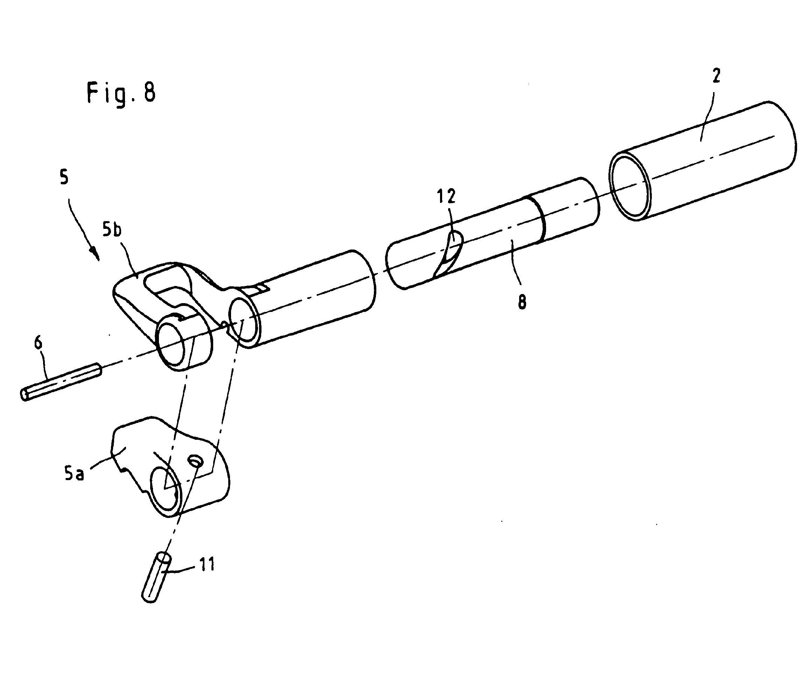

Fig. 8 zeigt eine alternative Ausführungsform zur Ausbildung des Übertragungshebels 7 zur Kopplung des Zug-/Druckelements 8 mit dem verschwenkbaren Maulteil 5a. Gemäß dieser alternativen Ausgestaltungsform ist der Übertragungshebel als fest mit dem verstellbaren Maulteil 5a verbundener Stift 11 ausgebildet, der in eine entsprechende Führungsbahn 12 im Zug-/Druckelement 8 eingreift. Bei einer Bewegung des Zug-/Druckelements 8 in Längsrichtung bewegt sich der senkrecht zur Längsrichtung des Schaftes 2 angeordnete Stift 11 in der Führungsbahn 12, die so geformt ist, dass sie so gebogen ist, dass das verschwenkbare Maulteil 5a durch den in der Führungsbahn 12 laufenden Stift 11 geöffnet und geschlossen werden kann. Die Übersetzung Verstellkraft-Maulteilkraft (Stanz- oder Schneidkräfte) kann bei dieser Ausführungsform durch entsprechendes Anpassen der Bahnkurve der Führungsbahn 12 so angepasst werden, dass die Übersetzung entweder über die gesamte Bahnkurve konstant ist oder aber veränderlich einstellbar ist.

Das Betätigen des in den Abbildungen Fig. 1 bis 7 dargestellten medizinischen Instruments 1 geschieht wie folgt:

Zum sicheren Ergreifen der Griffteile 3a, 3b der Handhabe 3 weisen diese an ihren freien Enden Fingerösen 3c auf. Durch das Verschwenken des Griffteils 3b um die Schwenkachse 4 wird das mit diesem Griffteil 3b gekoppelte Zug-/Druckelement 8 in Längsrichtung des Schaftes 2 verlagert. Zur Übertragung dieser translatorischen Bewegung des Zug-/Druckelements 8 auf das verschwenkbare Maulteil 5a sind bei dem dargestellten Ausführungsbeispiel das Zug-/Druckelement 8 und das verschwenkbare Maulteil 5a über den Übertragungshebel 7 miteinander gekoppelt. Um eine die Bewegung blockierende Totstellung zu vermeiden, ist der Übertragungshebel 7, wie aus Fig. 3 und 4 ersichtlich, in Richtung auf das Werkzeug 5 abgewinkelt angeordnet.

Alternativ zu der dargestellten Ausführungsform ist es auch möglich, den Übertragungshebel 7 so anzuordnen, dass er im wesentlichen in die entgegengesetzte Richtung zum Zug-/Druckelement 8 verläuft, also nach proximal abgewinkelt ist, was sich insbesondere für ebenso nach proximal abgewinkelte Werkzeuge 5 eignet. Bei dieser Ausgestaltungsform, bei der die Maulteile 5a, 5b des Werkzeugs 5 zum proximalen Ende des medizinischen Instruments 1 weisend gegenüber der Instrumentenlängsachse abgewinkelt sind, bewirkt die Abwinklung des Übertragungshebels 7, dass das Werkzeug 5 beim Schließen der Griffteile 3a, 3b der Handhabe 3 geöffnet wird, also umgekehrt zur üblichen Arbeitsweise arbeitet.

Durch eine Verlagerung des Angriffspunktes des Zug-/Druckelements 8 am verschwenkbaren Griffteil 3b von der der Hand des Operierenden zugewandten Seite bezüglich der Schwenkachse 4 hin zur der Hand des Operierenden abgewandten Seite bzw. umgekehrt, lässt sich auch bei einem vom Werkzeug 5 weg abgewinkelten Übertragungshebel 7 die Betätigung des Werkzeugs 5 wieder so einstellen, dass das Schließen der Griffteile 3a, 3b der Handhabe 3 ein Schließen des Werkzeugs 5 bewirkt.

Über das Zug-/Druckelement 8 und den Übertragungshebel 7 ist es so möglich, das rechtwinklig zur Längsachse des Instrumentenschaftes angeordnete Werkzeug 5 durch Betätigung des verschwenkbaren Handgriffs 3b zwischen einer geschlossenen Stellung der Maulteile 5a, 5b (Fig. 1 und 3) und einer geöffneten Stellung der Maulteile 5a, 5b (Fig. 2 und 4) zu verstellen.

### Bezugszeichenliste

- 1: medizinisches Instrument
- 2: Schaft
- 3: Handhabe
- 3a: starres Griffteil
- 3b: verschwenkbares Griffteil
- 3c: Fingeröse
- 4: Schwenkachse
- 5: Werkzeug
- 5a: verschwenkbares Maulteil
- 5b: starres Maulteil
- 6: Schwenkachse
- 7: Übertragungshebel
- 8: Zug-/Druckelement
- 9: Gelenkkugel
- 10: Kugelaufnahme
- 10a: Lagerelement
- 10b: Lagerelement
- 10c: Lagerelement
- 11: Stift
- 12: Führungsbahn

## Patentansprüche

1. Medizinisches Instrument mit einem hohlen Schaft (2), an dessen proximalem Ende eine aus mindestens zwei Griffteilen (3a, 3b) bestehende Handhabe (3) angeordnet ist und an dessen distalem Ende ein aus mindestens zwei Maulteilen (5a, 5b) bestehendes Werkzeug (5) angeordnet ist, dessen Maulteile (5a, 5b) des Werkzeugs (5) seitlich abgewinkelt zur Längsachse des Schaftes (2) ausgerichtet sind, wobei mindestens ein Maulteil (5a) des Werkzeugs (5) zum Öffnen und Schließen über ein verschwenkbar ausgebildetes Griffteil (3b) der Handhabe (3) gegenüber dem mindestens einen anderen Maulteil (5b) des Werkzeugs (5) verstellbar ist und das mindestens eine verstellbare Maulteil (5a) und das entsprechende, zum Verstellen des Maulteils (5a) dienende Griffteil (3b) der Handhabe (3) über ein in dem hohlen Schaft (2) gelagertes Zug-/Druckelement (8) miteinander verbunden sind, und wobei das Zug-/Druckelement (8) und das jeweilige Maulteil (5a) des Werkzeugs (5) über einen Übertragungshebel (7) miteinander verbunden sind,
**dadurch gekennzeichnet,**
**dass** der Übertragungshebel (7) als zweiseitig verdrehbar gelagerter Übertragungshebel (7) ausgebildet ist, der an beiden freien Enden Gelenkkugeln (9) aufweist, die jeweils in entsprechenden Kugelaufnahmen (10) am verstellbaren Maulteil (5a) und am Zug-/Druckelement (8) gelagert sind.

2. Medizinisches Instrument nach Anspruch 1, **dadurch gekennzeichnet, dass** die am verstellbaren Maulteil (5a) gelagerte Gelenkkugel (9) und/oder die am Zug-/Druckelement (8) gelagerte Gelenkkugel (9) aus zwei Halbkugeln mit unterschiedlichen Durchmessern besteht.

3. Medizinisches Instrument nach Anspruch 1 oder 2, **dadurch gekennzeichnet, dass** die Kugelaufnahme (10) am verstellbaren Maulteil (5a) aus ein oder zwei in das Maulteil (5a) einsetzbaren Lagerelementen (10a, 10b) besteht.

4. Medizinisches Instrument nach einem der Ansprüche 1 bis 3, **dadurch gekennzeichnet, dass** die Kugelaufnahme (10) am Zug-/Druckelement (8) ein in das Zug-/Druckelement (8) einsetzbares Lagerelement (10c) aufweist.

5. Medizinisches Instrument nach Anspruch 1, **dadurch gekennzeichnet, dass** der Übertragungshebel (7) in Richtung auf das Werkzeug (5) abgewinkelt ausgerichtet ist.

6. Medizinisches Instrument mit einem hohlen Schaft (2), an dessen proximalem Ende eine aus mindestens zwei Griffteilen (3a, 3b) bestehende Handhabe (3) angeordnet ist und an dessen distalem Ende ein aus mindestens zwei Maulteilen (5a, 5b) bestehendes Werkzeug (5) angeordnet ist, dessen Maulteile (5a, 5b) des Werkzeugs (5) seitlich abgewinkelt zur Längsachse des Schaftes (2) ausgerichtet sind, wobei mindestens ein Maulteil (5a) des Werkzeugs (5) zum Öffnen und Schließen über ein verschwenkbar ausgebildetes Griffteil (3b) der Handhabe (3) gegenüber dem mindestens einen anderen Maulteil (5b) des Werkzeugs (5) verstellbar ist und das mindestens eine verstellbare Maulteil (5a) und das entsprechende, zum Verstellen des Maulteils (5a) dienende Griffteil (3b) der Handhabe (3) über ein in dem hohlen Schaft (2) gelagertes Zug-/Druckelement (8) miteinander verbunden sind, und wobei das Zug-/Druckelement (8) und das jeweilige Maulteil (5a) des Werkzeugs (5) über einen Übertragungshebel (7) miteinander verbunden sind,
**dadurch gekennzeichnet,**
**dass** der Übertragungshebel (7) als fest mit dem verstellbaren Maulteil (5a) oder fest mit dem Zug-/Druckelement (8) verbundener Stift (11) ausgebildet ist, der in eine entsprechende Führungsbahn (12) im jeweils anderen Bauteil, dem Zug-/Druckelement (8) oder dem verstellbaren Maulteil (5a), eingreift.

7. Medizinisches Instrument nach einem der Ansprüche 1 bis 6, **dadurch gekennzeichnet, dass** das Zug-/Druckelement (8) als Zug-/Druckstange ausgebildet ist.

## Claims

1. Medical instrument with a hollow shaft (2) which, at its proximal end, has a handle (3) consisting of at least two grip parts (3a, 3b), and, at its distal end, has a tool (5) consisting of at least two jaw parts (5a, 5b), the jaw parts (5a, 5b) of the tool (5) being oriented laterally at an angle with respect to the longitudinal axis of the shaft (2), where at least one jaw part (5a) of the tool (5) can be adjusted relative to the at least one other jaw part (5b) of the tool (5) for purposes of opening and closing via a pivotable grip part (3b) of the handle (3), and the at least one adjustable jaw part (5a) and the corresponding grip part (3b) of the handle (3) that serves to adjust the jaw part (5a) are connected to one another via a pull/push element (8) mounted in the hollow shaft (2), and where the pull/push element (8) and the respective jaw part (5a) of the tool (5) are connected to one another via a transmission lever (7), **characterized in that** the transmission lever (7) is designed as a transmission lever (7) which is pivotably mounted at both ends and which, at both free ends, has balls (9) that are each mounted in corresponding ball seats (10) on the adjustable jaw part (5a) and on the pull/push element (8).

2. Medical instrument according to Claim 1, **characterized in that** the ball (9) mounted on the adjustable jaw part (5a) and/or the ball (9) mounted on the pull/push element (8) consists of two half-balls with different diameters.

3. Medical instrument according to Claim 1 or 2, **characterized in that** the ball seat (10) on the adjustable jaw part (5a) consists of one or two bearing elements (10a, 10b) that can be fitted into the jaw part (5a).

4. Medical instrument according to one of Claims 1 to 3, **characterized in that** the ball seat (10) on the pull/push element (8) has a bearing element (10c) that can be fitted into the pull/push element (8).

5. Medical instrument according to Claim 1, **characterized in that** the transmission lever (7) is oriented at an angle in the direction of the tool (5).

6. Medical instrument with a hollow shaft (2) which, at its proximal end, has a handle (3) consisting of at least two grip parts (3a, 3b), and, at its distal end, has a tool (5) consisting of at least two jaw parts (5a, 5b), the jaw parts (5a, 5b) of the tool (5) being oriented laterally at an angle with respect to the longitudinal axis of the shaft (2), where at least one jaw part (5a) of the tool (5) can be adjusted relative to the at least one other jaw part (5b) of the tool (5) for purposes of opening and closing via a pivotable grip part (3b) of the handle (3), and the at least one adjustable jaw part (5a) and the corresponding grip part (3b) of the handle (3) that serves to adjust the jaw part (5a) are connected to one another via a pull/push element (8) mounted in the hollow shaft (2), and where the pull/push element (8) and the respective jaw part (5a) of the tool (5) are connected to one another via a transmission lever (7), **characterized in that** the transmission lever (7) is designed as a pin (11) (7) which is fixedly connected to the adjustable jaw part (5a) or fixedly connected to the pull/push element (8) and which engages in a corresponding guide track (12) in the respective other component, namely the pull/push element (8) or the adjustable jaw part (5a).

7. Medical instrument according to one of Claims 1 to 6, **characterized in that** the pull/push element (8) is designed as a pull/push rod.

## Revendications

1. Instrument médical comportant une tige (2) creuse, sur l'extrémité proximale de laquelle est agencé un manche (3) formé par au moins deux éléments de préhension (3a, 3b), et sur l'extrémité distale de laquelle est agencé un outil (5) formé par au moins deux éléments de mâchoire (5a, 5b), les éléments de mâchoire (5a, 5b) de l'outil (5) étant orientés latéralement sous forme coudée par rapport à l'axe longitudinal de la tige (2), au moins un élément de mâchoire (5a) de l'outil (5) pouvant être déplacé par rapport audit au moins un autre élément de mâchoire (5b) de l'outil (5)pour l'ouverture et la fermeture, par l'intermédiaire d'un élément de préhension (3b) pivotant du manche (3), et ledit au moins un élément de mâchoire (5a) mobile et l'élément de préhension (3b) correspondant du manche (3), pour le déplacement de l'élément de mâchoire (5a), étant reliés l'un à l'autre par l'intermédiaire d'un élément de traction et pression (8) monté dans la tige (2) creuse, et l'élément de traction et pression (8) et l'élément de mâchoire (5a) concerné de l'outil (5) étant reliés entre eux par l'intermédiaire d'un levier de transmission (7),
**caractérisé en ce que** le levier de transmission (7) est réalisé sous la forme d'un levier de transmission (7) monté rotatif des deux côtés, comportant sur les deux extrémités libres des rotules (9), qui sont montées dans des logements sphériques (10) correspondants sur l'élément de mâchoire (5a) mobile et sur l'élément de traction et pression (8).

2. Instrument médical selon la revendication 1, **caractérisé en ce que** la rotule (9), montée sur l'élément de mâchoire (5a) mobile, et/ou la rotule (9), montée sur l'élément de traction et pression (8), sont formées par deux demi-sphères avec des diamètres différents.

3. Instrument médical selon la revendication 1 ou 2, **caractérisé en ce que** le logement sphérique (10) sur l'élément de mâchoire (5a) mobile est formé par un ou deux éléments de palier (10a, 10b) insérables dans l'élément de mâchoire (5a).

4. Instrument médical selon l'une quelconque des revendications 1 à 3, **caractérisé en ce que** le logement sphérique (10) sur l'élément de traction et pression (8) comporte un élément de palier (10c) insérable dans l'élément de traction et pression (8).

5. Instrument médical selon la revendication 1, **caractérisé en ce que** le levier de transmission (7) est orienté en étant coudé vers l'outil (5).

6. Instrument médical comportant une tige (2) creuse, sur l'extrémité proximale de laquelle est agencé un manche (3) formé par au moins deux éléments de préhension (3a, 3b), et sur l'extrémité distale de laquelle est agencé un outil (5) formé par au moins deux éléments de mâchoire (5a, 5b), les éléments de mâchoire (5a, 5b) de l'outil (5) étant orientés latéralement sous forme coudée par rapport à l'axe longitudinal de la tige (2), au moins un élément de mâchoire (5a) de l'outil (5) pouvant être déplacé par rapport audit au moins un autre élément de mâchoire (5b) de l'outil (5), pour l'ouverture et la fermeture, par l'intermédiaire d'un élément de préhension (3b) pivotant du manche (3), et ledit au moins un élément de mâchoire (5a) mobile et l'élément de préhension (3b) correspondant du manche (3), pour le déplacement de l'élément de mâchoire (5a), étant reliés l'un à l'autre par l'intermédiaire d'un élément de traction et pression (8) monté dans la tige (2) creuse, et l'élément de traction et pression (8) et l'élément de mâchoire (5a) concerné de l'outil (5) étant reliés entre eux par l'intermédiaire d'un levier de transmission (7),
**caractérisé en ce que** le levier de transmission (7) est réalisé sous la forme d'un tenon (11), qui est relié de manière fixe à l'élément de mâchoire (5a) mobile ou de manière fixe à l'élément de traction et pression (8), et qui s'engage dans une voie de guidage (12) correspondante, agencée dans respectivement l'autre partie, à savoir l'élément de traction et pression (8) ou l'élément de mâchoire (5a) mobile.

7. Instrument médical selon l'une quelconque des revendications 1 à 6, **caractérisé en ce que** l'élément de traction et pression (8) est réalisé sous la forme d'une tige de traction et pression.
